# EUROPEAN PATENT APPLICATION

(11) **EP 4 205 647 A1**
(43) Date of publication of application: **05.07.2023**
(21) Application number: 21383241.3
(22) Date of filing: 30.12.2021
(51) Int. Cl.: A61B 5/145, A61B 5/00, A61B 5/0205

(54) **A WEARABLE DEVICE FOR CONTINUOUS MONITORING OF HEALTH PARAMETERS**

(71) Applicant: Onalabs Inno-Hub, 08012 Barcelona (ES)
(72) Inventor: RABOST GARCIA, Genís, 08012 Barcelona (ES); MUÑOZ PASCUAL, Francesc Xavier, 08012 Barcelona (ES); AGUILAR TORÁN, Javier, 08012 Barcelona (ES); PUNTER VILLAGRASA, Jaime, 08012 Barcelona (ES); COLMENA RUBIAL, Valeria, 08012 Barcelona (ES)
(74) Representative: Hoffmann Eitle

(57) **Abstract**

The present invention refers to a wearable device for the continuous monitoring of health condition of patients or sport persons without the need of blood extraction. The device comprises: a housing having at least part of a processing unit enclosed therein, means for attaching the main housing to a part of the user's body, and a consumable component configured to be manually coupled and uncoupled with the main housing. The consumable component comprises: a sweat collection inlet for collecting sweat when the device is worn by the user, at least one sensor for measuring a sweat biomarker. The processing unit is adapted for receiving and processing data provided by the sensor. The invention can advantageously be used in sports medicine and/or sports health sectors for remote effort and/or fatigue assessment.

## Description

### Field and object of the invention

The present invention belongs to the technical filed of wearable medical devices both for clinical and sport applications.

An object of the invention, is the provision of a wearable device for monitoring of health condition of patients or sport persons, without blood extraction, that can be used for a large variety of applications, and which can be used in a simple and intuit manner.

The invention can advantageously be used in sports medicine and/or sports health sectors for remote effort and/or fatigue assessment.

### Background of the invention

There are many types of wearable devices that can be worn by a user to continuously monitor an individual's activities, such as walking or running without unduly interrupting or limiting those activities. These wearable devices include electronics, and physiological sensors configured to sense certain physiological parameters of the wearer, such as heart rate, as well as motion sensors, GPS, etc.

These known devices generally have the same format, and are adapted to monitor only one physiological parameter of the wearer, and have limited capacities in terms of electronic processing, and communications capacities.

### Summary of the invention

The invention refers to a wearable device for continuous monitoring of health parameters of a user, that comprises three main parts, namely: a housing, means for attaching the housing to a part of the user's body, and a fungible or consumable component configured to be manually attachable and detachable from the housing, and that can be disposed after its use.

The consumable component comprises: a sweat collection inlet for collecting sweat when the device is worn by the user, at least one sensor for measuring a sweat biomarker, and a microfluidic channel for conveying collected sweat from the inlet to the sweat sensor.

Preferably, the consumable component has a sensing chamber communicated with the sweat inlet through the microfluidic channel. The sensing chamber encloses at least one of the following sensors: a sweat lactate sensor, a sweat conductivity sensor, a metabolites sensor, an ions sensor, and/or an amino acids sensor.

The advantage of this format of wearable device formed by a permanent part (the housing with the electronics of the device), and a consumable part with the sensors that can be disposed after its use, is that the same device can be used for a large variety of health monitoring applications, simply by providing a set of consumables each one provided with the sensors required for each particular application.

In addition, the wearable device comprises a processing unit enclosed in the housing and adapted for processing data provided by the sensor or sensors, and electric connection means for electrically connecting the sensor or sensors with the processing unit, when the consumable component is operatively attached to the main housing.

Preferably, the wearable device includes a communication module enclosed in the housing, and adapted for the wireless transmission of data processed and calculated by the processing unit, to an external device such as: a smart phone, a smart watch, etc.

The housing has a front surface and a back surface, and the consumable component is attachable to the back surface of the housing, in a way that the consumable component is overlapped with the back surface of the housing when they are operatively attached.

In turn, the consumable component has a contact surface provided to be in contact with the user's skin when the consumable component is operatively attached to the housing and the housing is attached to a part of the user's body. The sweat collection inlet is provided on the consumable component's contact surface, to collect sweat from the user's skin.

The housing has a cavity at its back surface for receiving a battery for supplying power to the processing unit and to other electronics of the device, and a lid for closing the cavity and enclosing the battery therein, so that for replacing the battery the lid has to be removed. The device is configured such that the consumable component is placed over the lid, when the consumable component is operatively attached to the housing.

The means for attaching the housing to a part of the user's body, are preferably implemented as a flexible band or strap having two ends respectively couplable with the housing. Preferably, the flexible band is fitted with at least one biosensor for measuring a vital-sign or physiological sign of the user, when the flexible band is attached to a part of the user's body such as an arm, wrist, chest etc., thus, in addition to the sweat measurements, a vital sign or signs measurements are also taken in direct contact with the skin, with the same de device, and simultaneously while sweat parameters are measured. The biosensor or biosensors are selected from the following list: a heart rate sensor, a respiratory rate sensor, blood pressure sensor, body temperature sensor, and oxygen saturation sensor.

Alternatively, the means for attaching the main housing to a part of a user's body, comprises an adhesive surface suitable to be adhered on a user's skin. For example, this adhesive surface is provided on a surface of the consumable component meant to be in contact with the user's skin.

The device has a pair of connectors for mechanically connecting the flexible band with the housing, and for electrically connecting the biosensors with the processing unit.

Preferably, the consumable component is generally a flat body, in the form of a card, so that it does not protrude from the housing when they are coupled.

A part or area of the back surface of the housing is generally flat, and the consumable component is attachable and detachable from the housing by moving the consumable component on a plane coplanar with said generally flat part of the back surface. This way of coupling the consumable component with the housing, provides the advantage that it is easy and intuitive for users to attach and detach both parts even when the device is worn by a user.

In a preferred embodiment, for attaching the consumable component to the housing, the housing has at its back surface a pair of guides opposite each other, and the consumable component has a pair of sides wings dimensioned and configured such that the consumable component fits inside the space defined in between the pair of guides. The consumable component is attachable and detachable from the housing, by sliding its side wings on the guides and by moving the consumable component on the back surface of the housing.

When coupled, the consumable component is retained firmly attached to the housing, while the user is running or working out.

It would be understood by a skilled person in the art, that the means for attaching and detaching the consumable component from the housing, can be embodied in many different ways within the scope of this invention.

Preferably, the consumable component further comprises a sweat volume sensor for measuring volume of the collected sweat, and the processing unit is adapted for receiving and processing data provided by the sweat biomarker sensor, the vital-sign biosensor of the flexible band, and the sweat volume sensor.

The sweat volume sensor comprises a microfluidic circuit or a microfluidic reservoir, fluidly communicated with the sensing chamber and arranged downstream the sensing chamber, and a pair of electrodes such that the microfluidic reservoir is arranged in between the two electrodes, in a way that a capacitance value between the two electrodes is variable depending on the amount of sweat in the reservoir. The two electrodes are a pair of conductive strips opposite each other, and more preferably the electrodes are embodied as conductive flexible strips.

The microfluidic reservoir can have any form, for example the microfluidic reservoir can be a straight or curved conduit, or it can be formed as a conduit having a meander configuration.

In a preferred embodiment, the sweat sensor is a sweat lactate sensor, and the vital-sign sensor is a heart rate sensor, and wherein the processing unit is further adapted to calculate or estimate, preferably by means of machine learning algorithms, a blood lactate concentration based on data provided by: the sweat lactate sensor, the sweat volume sensor and heart rate sensor. This data received by the processing unit is in the form of electric signals.

In summary, some of the main advantages of the invention are the followings:
- the format of the wearable device, is already used by athletes (heart rate monitor) to which they are accustomed in their regular training;
- the permanent part of the device (housing) can be used by itself, in case that chemical measurement with the consumable part is not necessary (already known training routine, etc...);
- the permanent part (housing) is compatible with different types of consumable parts depending on the parameters to be measured, such that the device is an useful tool not only for the end user but also for the clinic/sports physician, who can adapt the device to the requirements of the patient/study;
- simple to use by insertion of the consumable that guarantees placement in the same place every time, limiting the variability that the user's own operation may entail;
- it allows to have more advanced electronics with advanced functionalities compared to patch wearables such as battery management, data processing, flash memory in case there is no possibility of communicating the data;
- the consumable part might have an adhesive surface to enhance its attachment with the permanent part.

The main application of the invention is focused on sports medicine or sports health sector, for example for monitoring dehydration monitoring in athletes, but depending on the combination of sensors used, the product can be oriented to different applications.

### Brief description of the drawings

Preferred embodiments of the invention are henceforth described with reference to the accompanying drawings, wherein:
Figure 1.- shows a perspective view of a preferred embodiment of the invention including an attachment band or strap.
Figure 2.- shows a perspective view of the main housing and the consumable part partially coupled together.

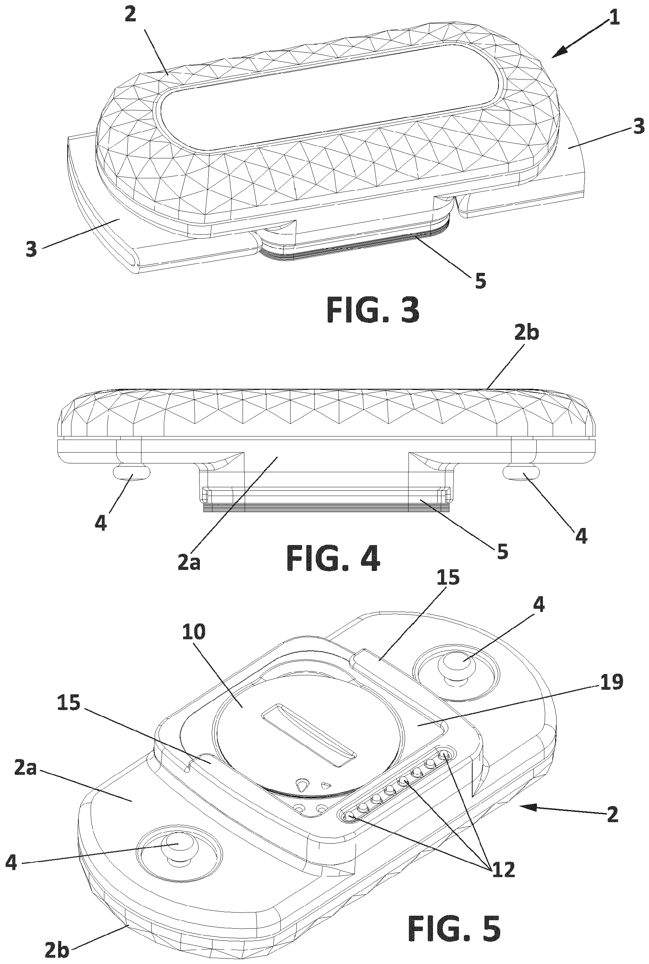

Figure 3.- shows another perspective view of the main housing from above, and part of the band ends.
Figure 4.- shows a front elevational view of the main housing.
Figure 5.- shows a perspective view of the main housing from below.
Figure 6.- shows an exploded view of the main housing components.
Figure 7.- shows in Figure A a perspective view of the main housing from below. In Figure B with the consumable part partially coupled, and in Figure C with the consumable part fully coupled.
Figure 8.- shows an schematic representation of the sweat volume sensor. The figure represents four stages (A - B) of filling of the microfluidic channel.
Figure 9.- shows a perspective view of the consumable device from the face opposite the surface meant to be in contact with the skin.
Figure 10.- shows a schematic representation in plan view of the consumable device.
Figure 11.- shows a cross-sectional view of the consumable device, taken at cutting plane A-A in figure 10.
Figure 12.- shows another cross-sectional view of the consumable device, taken at cutting plane B-B in figure 10.

### Preferred embodiment of the invention

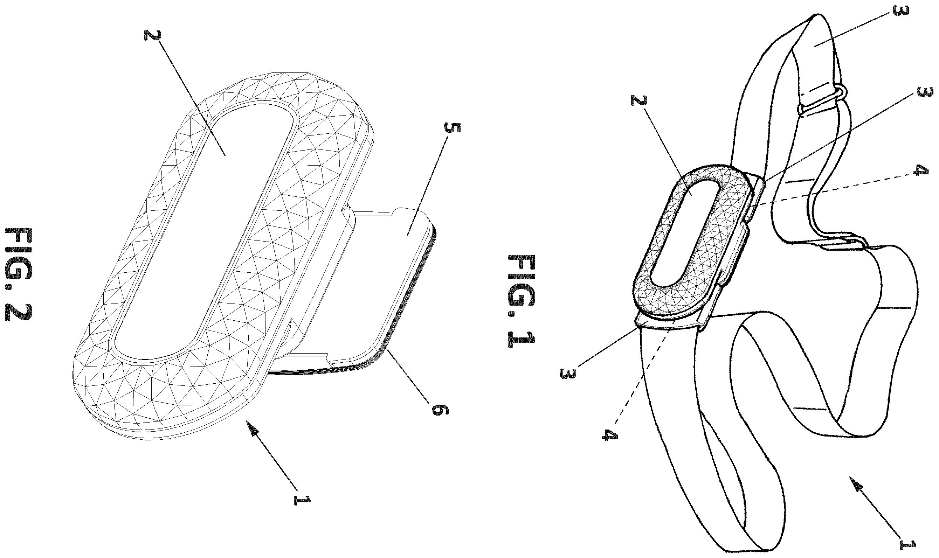

**Figure 1** shows an exemplary embodiment of a wearable device (1) according to the invention comprising a housing (2) and a flexible band or strap (3) for attaching the main housing to a part of a user's body, and a pair of electric and mechanic connectors or snap buttons (4) provided in the housing (2) for mechanically and electrically connecting the flexible band ends with the housing (2).

The flexible band (3) is implemented as an elastic textile tape with female connectors at its ends to be coupled with the snap buttons (4). The flexible band (3) is fitted conventionally with at least one biosensor for measuring a vital-sign or physiological sign of the user, in a known manner, for example a pair of electrodes to measure heart rate. The electrodes are made of bioelectric silicone to capture the electrocardiogram signal and extract the heart rate, and they have sufficient conductivity to obtain a quality heart rate signal and are resistant to continuous use and washing.

As shown more clearly in **Figure 2****,** the device (1) includes a consumable component (5) configured to be manually attached and detached from the housing (2), and having a contact surface (6) provided to be in contact with the user's skin when the consumable component (5) is coupled with the housing (2), and the housing (2) is attached to a user's body by means of the flexible band (3).

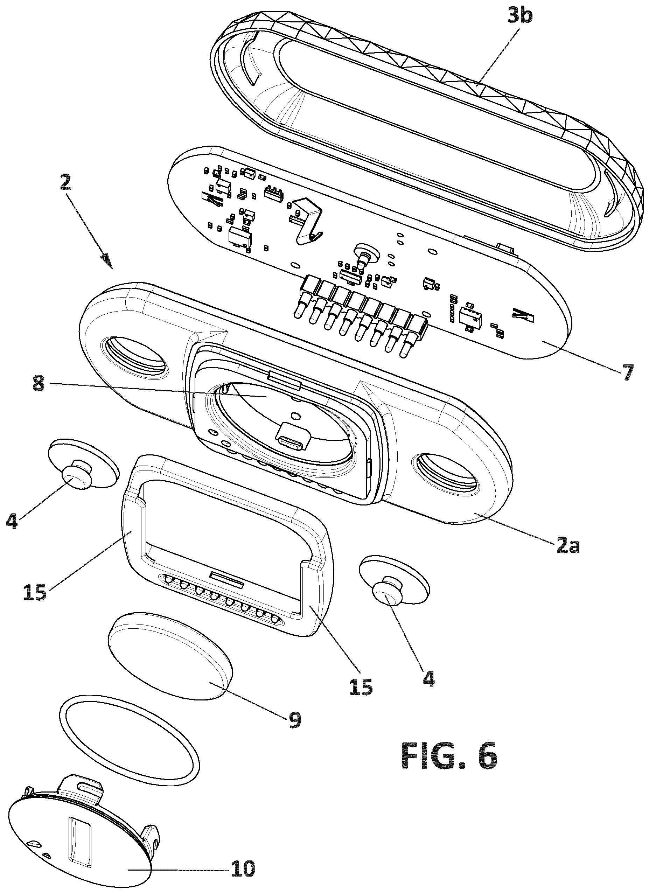

As shown in **Figure 6****,** the housing (2) is formed by two couplable parts, a base (2a) and a cover (2b) that configure a space when they are coupled within which an electronic circuit (7) is enclosed. This electronic circuit (7) implements the processing unit, sensors instrumentation, battery management, data processing and communication module for the wireless transmission of data processed by the processing unit.

The housing (2) in particular the base (2a) has a recess or cavity (8) for receiving a battery (9) for supplying power to the electronic circuit (7), and a lid (10) for closing and opening the cavity (8).

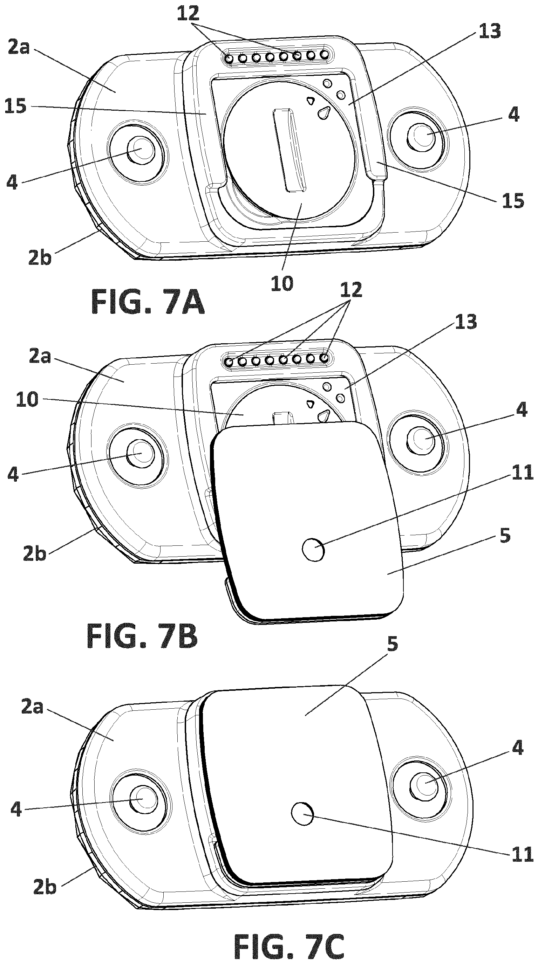

As represented in **Figures 2****,** **7B** and **7C****,** the consumable component (5) is generally a flat body, and the housing (2) and the consumable component (5) are configured, such that the consumable component (5) is couplable with the housing (2) by moving the consumable component (5) on a plane coplanar to the generally flat back surface (19) of the housing (2).

With this arrangement and as shown in **Figure 7C****,** the consumable component (5) is placed over lid (10) when is fully attached to the housing (2), and the lid (10) can only be accessed when the consumable component (5) is taken out from the housing (2).

A set of electric connectors (12) are provided at the back surface (13) of the base (2a) of the housing (2), in order to electrically connect the sensors fitted in the consumable component (5) with the processing unit (7). These connectors (12) are known spring-biased connectors, that stablish electric contact with corresponding electric pads (17) (provided in the consumable component (5), in a known manner when this is coupled with the housing (2).

The consumable component (5) can be mechanically and electrically coupled and uncoupled from the housing (2), in a quick and intuitive manner for a user, while ensuring functionality and good electrical connection with the housing (2).

The housing (2) has a back surface (13) provided with a pair of guides (15) opposite each other, in a way that a space or pocket is formed in between the guides (15) for receiving the consumable component (5) therein. In turn, the consumable component (5) has a pair of sides wings (16), dimensioned and configured to fit in the space formed in between the guides (15), such that the consumable component (5) is attachable to the housing (2) by inserting its side wings (16) respectively in the guides (15), and by moving the consumable component on the back surface (13) of the housing (2), as represented in the sequence of **Figures 7A, 7B,** and **7C****.**

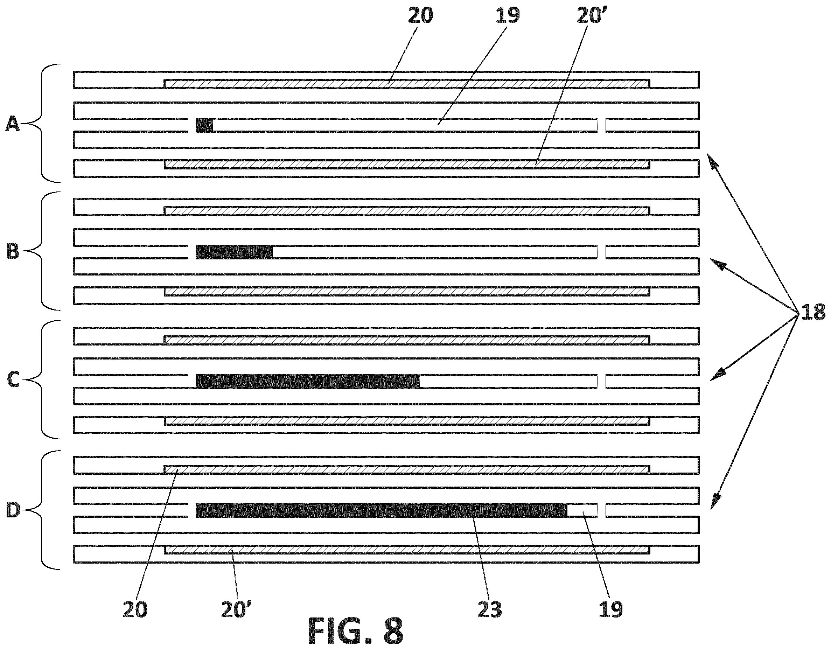

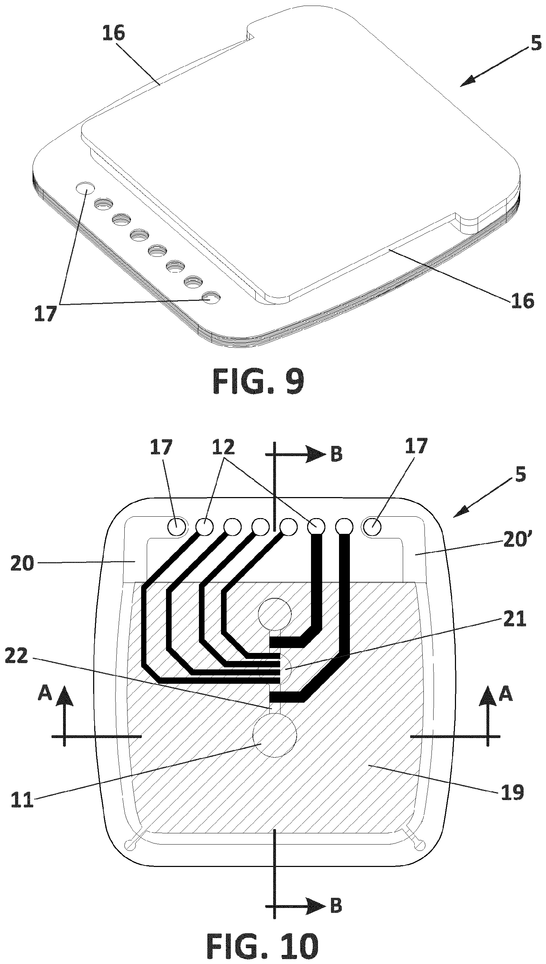

The consumable component (5) has a sweat volume sensor (18) represented schematically in **Figure 8****,** which comprises a microfluidic circuit or a microfluidic reservoir (19), fluidly communicated with a sensing chamber (21) and arranged downstream the sensing chamber (21), such that sweat would enter the inlet (11), and flow along the microfluidic channel (22) and the sensing chamber (21) to gradually fill the reservoir (19), as more clearly represented in **Figure 10****.**

In addition, the sweat volume sensor (18) comprises a pair of electrodes (20,20') and the microfluidic reservoir (19) arranged in between the two electrodes, such that a capacitance value between the two electrodes is variable depending on the amount of sweat in the reservoir.

The consumable component (5) can be manufactured as a stack of layers of plastic materials where the microfluidic channels and sensing chamber are formed by laser cutting or die cutting. The integrated sensors are electrochemical in nature, and therefore require electrodes that are fabricated by screen printing.

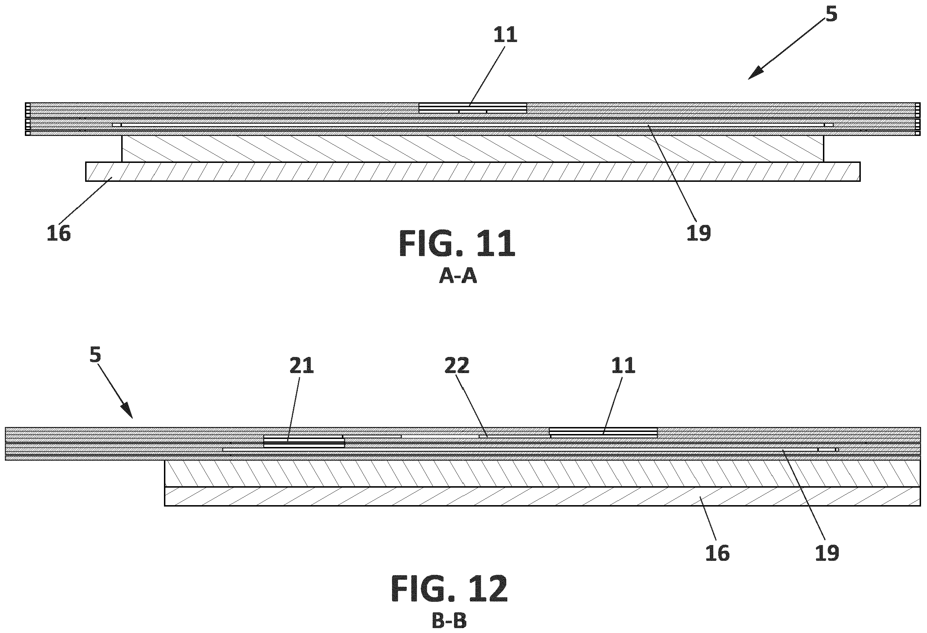

As shown in **Figures 11** and **12****,** in this particular embodiment, the sweat inlet (11), the microfluidic channel (22) and the sensing chamber (21), are placed above the microfluidic reservoir (19).

## Claims

1. A wearable device for continuous monitoring of health parameters of a user, comprising:
a housing having a front surface and a back surface, and a processing unit enclosed therein,
means for attaching the housing to a part of the user's body,
a consumable component configured to be manually coupled and uncoupled with the housing, and having a contact surface provided to be in contact with the user's skin when the consumable component is operatively coupled with the housing and the housing is attached to a part of the user's body, and
wherein the consumable component comprises:
a sweat collection inlet formed in the consumable component's contact surface, for collecting sweat when the device is worn by the user,
at least one sensor for measuring a sweat biomarker,
a microfluidic channel for conveying collected sweat from the inlet to the sweat
sensor, and
wherein the wearable device further comprises:
electric connection means for electrically connecting the sensor with the processing unit, when the consumable component is operatively coupled with the housing, and
wherein the processing unit is adapted for processing data provided by the sensor.

2. A wearable device according to claim 1, wherein a part of the back surface of the housing is generally flat, and wherein the consumable component is couplable and un-couplable with the housing by moving the consumable component on a plane parallel to said generally flat part of the back surface, or on a plane coplanar with the flat surface.

3. A wearable device according to claim 1 or 2, wherein the consumable component is generally a flat body.

4. A wearable device according to any of the preceding claims, wherein the means for attaching the main housing to a part of a user's body, comprises a flexible band having two ends respectively couplable with the housing.

5. A wearable device according to any of the claims 1 to 3, wherein the means for attaching the main housing to a part of a user's body, comprises an adhesive surface suitable to be adhered on a user's skin, and wherein preferably the adhesive surface is provided on the consumable component's contact surface.

6. A wearable device according to any of the preceding claims, wherein the housing has a pair of guides opposite each other, and wherein the consumable component has a pair of sides wings, and wherein the housing and the consumable part are configured, such that the consumable component is couplable with the housing by inserting its side wings respectively in the guides and by moving the consumable component on the back surface of the housing.

7. A wearable device according to any of the preceding claims, wherein housing has a cavity at its back surface for receiving a battery for supplying power to the processing unit, and a lid for closing the cavity and enclosing the battery therein, and wherein the device is configured such that the consumable component is overlapped with the lid, when the consumable component is operatively coupled with the housing.

8. A wearable device according to claims 4 to 7, wherein the housing is provided by a pair of electric connectors, and each end of the flexible band is fitted with metallic connectors for mechanically and electrically connecting the flexible band and the biosensor with the pair of electric connectors of the housing.

9. A wearable device according to claim 4, wherein the flexible band is provided with at least one biosensor arranged for measuring a vital-sign or physiological sign of the user, when the device is worn by a user, and wherein the biosensor is selected from the following list: a heart rate sensor, a respiratory rate sensor, blood pressure sensor, body temperature sensor, and oxygen saturation sensor.

10. A wearable device according to claim 8, wherein the consumable component further comprises a sweat volume sensor for measuring volume of the collected sweat, and wherein the processing unit is adapted for receiving and processing data provided by the sweat sensor, the vital-sign biosensor and the sweat volume measuring device.

11. A wearable device according to any of the preceding claims, further comprising a communication module enclosed in the housing, and adapted for the wireless transmission of data processed by the processing unit.

12. A wearable device according to claim 9, wherein the sweat sensor is a sweat lactate sensor, and the vital-sign sensor is a heart rate sensor, and wherein the processing unit is further adapted to calculate or estimate, preferably by means of machine learning algorithms, a blood lactate concentration based on data provided by: the sweat lactate sensor, the sweat volume sensor and heart rate sensor.

13. A wearable device according to any of the preceding claims, further comprising sensing chamber and at least one of the following sensors: a sweat lactate sensor, a sweat conductivity sensor, metabolites sensor, ions sensors, amino acids sensor, and, placed in the sensing chamber, and wherein the microfluidic channel communicates the sweat inlet with the sensing chamber.

14. A wearable device according to any of the preceding claims, wherein the sweat volume sensor comprises a pair of electrodes and a microfluidic reservoir in between the pair of electrodes, and fluidly communicated with the sensing chamber and arranged downstream the sensing chamber, such that a capacitance value between the two electrodes is variable depending on the amount of sweat in the reservoir.
